# EUROPEAN PATENT SPECIFICATION

(11) **EP 4 250 990 B1**
(45) Date of publication and mention of the grant of the patent: **20.08.2025**
(21) Application number: 21810192.1
(22) Date of filing: 21.10.2021
(51) Int. Cl.: A61F 9/04, A41D 13/11

(54) **VISOR AND PROTECTIVE FACE SHIELD APPARATUS AND METHODS OF ASSEMBLY**
VISIER UND GESICHTSSCHUTZVORRICHTUNG SOWIE MONTAGEVERFAHREN DAFÜR
VISIÈRE ET APPAREIL D'ÉCRAN FACIAL DE PROTECTION ET PROCÉDÉS D'ASSEMBLAGE

(30) Priority: 26.11.2020 US 202017105577
(43) Date of publication of application: 04.10.2023
(73) Proprietor: OP-D-OP, Inc., Roseville, California 95678 (US)
(72) Inventor: LANDIS, Timothy J., Roseville, California 95678 (US)
(74) Representative: Lusinchi, Laurent Pierre
(86) International application number: PCT/US2021/056025
(87) International publication number: WO 2022/087247

(56) References cited:
- US-A- 1 333 102
- US-A- 2 004 098
- US-A- 4 864 653
- US-A- 4 920 576

## Description

### BACKGROUND

### 1. Technical Field

The technology of this invention pertains generally to personal protection devices that cover a portion of the user's face, and more particularly to a visor and face shield apparatus that provides protection to the wearer from exposure to germs, viruses other contaminants.

### 2. Background Discussion

Masks made from gauze and paper or from fabric materials are well known devices that can be worn by a person in an effort to prevent exposure to germs, viruses other contaminants and to prevent spread to others. However, wearing such masks can be hot and uncomfortable, and putting the masks on and removing them can be time-consuming and sometimes difficult. Furthermore, breath can condense within the mask and hence the mask can become saturated with moisture which results in the mask failing to be an effective barrier to viruses and bacteria. The condensation can also cause fogging of eyeglasses worn by the user. Furthermore, masks can cause the wearer to re-inhale exhaled breath causing the CO₂ content of the blood to rise. The result of this may be increased heart and respiration rates and higher body temperatures and perspiration.

Alternatives to conventional mask include visor and face shield devices such as those described in US 4 852 176 A, US 4 864 653 A, US 4 920 576 A, US 2 004 098 A and US 1 333 102 A Those devices address the problems with conventional masks but can be difficult to assemble by a user.

### BRIEF SUMMARY

This invention describes improved visor and protective face shield apparatuses and assembly mechanisms.

In one embodiment, a visor and protective face shield apparatus comprises (a) a transparent or non-transparent visor and (b) a transparent face shield. In this embodiment, the visor and face shield are separate components that are easily assembled using improved tabs and slots such that the face shield is supported by the visor. In one embodiment, the visor has arms which are adjustable to fit the head size of the wearer. In one embodiment, a forehead support member is provided.

In another embodiment, a visor and protective face shield apparatus comprises (a) a transparent or non-transparent visor and (b) a transparent face shield that is integral with the visor. In this embodiment, the visor and face shield are formed initially as a unitary flat blank. To assemble the apparatus, the face shield is bent with respect to the visor and secured into place using improved tabs and slots such that the visor supports the face shield. In one embodiment, the visor has arms which are adjustable to fit the head size of the wearer. In one embodiment, a forehead support member is provided.

In all embodiments, when the apparatus is assembled and placed on the wearer's head, the visor preferably supports the face shield and the face shield extends downward from the visor. The face shield also preferably extends down to below the level of the wearer's mouth and around the sides of the wearer's face, thus providing superior, frontal and lateral protection from contaminants. It will be appreciated that a face shield that is supported by a visor and which extends downward therefrom when assembled or worn can also be said to "depend" from the visor.

In one embodiment, the face shield is connected to the visor and secured into place using an innovative tab and slot mechanism. In one embodiment, the visor has an edge and a plurality of slots positioned distal of the edge. In one embodiment, the face shield has an edge and a plurality of tabs that extend from the edge of the face shield. The tabs and slots are configured such that a tab can easily be bent and inserted into a corresponding slot and, after, the tab is inserted, the automatically springs back to its original flat position to secure the face shield to the visor.

In one embodiment, the slots have a straight central portion and angled portions positioned at each end of the straight central portion. In one embodiment, the edge of the face shield is partially "undercut" beneath each tab. Undercuts span from the sides of the tabs toward the central axis through the tab wherein the tab appears "tree-shaped" with a trunk portion and adjacent side portions, with the "undercuts" forming notches in the face shield positioned beneath each of the side portions of the tab. This configuration facilitates greater ease of tab insertion and assembly of the apparatus and, further, provides for a more secure connection between the visor and the face shield.

Another aspect of the technology is that flat tabs with the undercuts facilitate ease of manufacturing.

Further aspects of the technology described herein will be brought out in the following portions of the specification, wherein the detailed description is for the purpose of fully disclosing preferred embodiments of the technology without placing limitations thereon.

### BRIEF DESCRIPTION OF THE SEVERAL VIEWS

### OF THE DRAWING(S)

The technology described herein will be more fully understood by reference to the following drawings which are for illustrative purposes only:
FIG. 1 is an assembled perspective view of a visor and protective face shield apparatus according to an embodiment of the technology presented herein.
FIG. 2 is an exploded view of the apparatus of FIG. 1.
FIG. 3 is a detail view of a cutout for retaining the forehead support member illustrated in FIG. 1 and FIG. 2.
FIG. 4 is a detail view of a connecting slot according to an embodiment of the technology presented herein.
FIG. 5 is a detail view of a connecting tab according to an embodiment of the technology presented herein.
FIG. 6 is a assembled bottom view of the forehead support member shown in FIG. 2.
FIG. 7 is a plan view of the visor and face shield of FIG. 1 and FIG. 2 prior to assembly and showing separate components, and showing a wide arm spacing according to an embodiment of the technology presented herein.
FIG. 8 is a plan view of the visor and face shield of FIG. 1 and FIG. 2 prior to assembly and showing separate components, and showing a narrow arm spacing according to an embodiment of the technology presented herein.
FIG. 9 is a closeup view of a slot according to an embodiment of the technology presented herein.
FIG. 10 through FIG. 12 are closeup views of different tab configurations according to embodiments of the technology presented herein.
FIG. 13 through FIG. 22 illustrate examples of different slot and tab configurations and dimensions according to embodiments of the technology presented herein.
FIG. 23 shows a plan view of a unitary visor and face shield blank with wide arm spacing along with a perspective view of visor and face shield assembled from the blank according to an embodiment of the technology presented herein.
FIG. 24 shows a plan view of a unitary visor and face shield blank with narrow arm spacing along with a perspective view of visor and face shield assembled from the blank according to an embodiment of the technology presented herein.

### DETAILED DESCRIPTION

### A. Visor and Face Shield Assembled From Separate Components

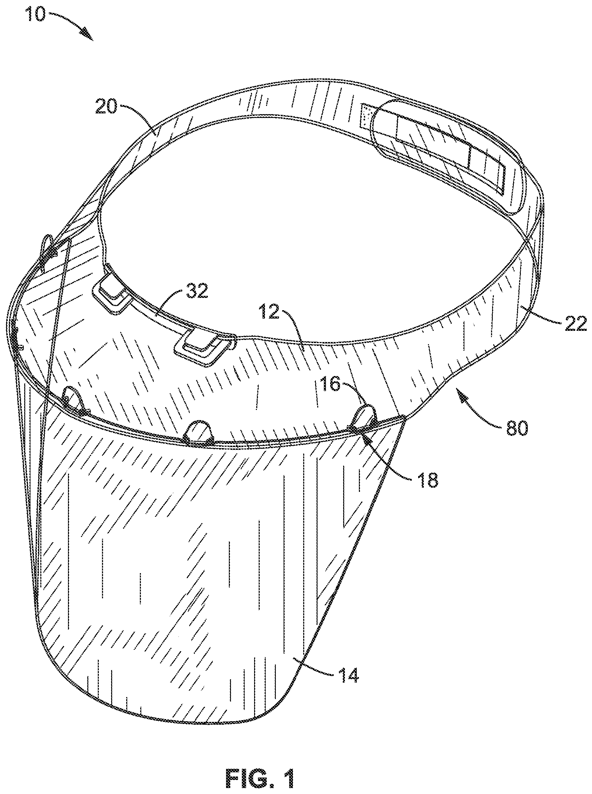

Referring first to FIG. 1 through FIG. 12, in one embodiment a visor and protective face shield apparatus 10 comprises a transparent, non-transparent, or tinted visor 12 and a transparent or tinted face shield 14. In this embodiment, the visor and face shield are separate components that are easily assembled using improved tabs 16 and slots 18 such that the face shield is supported by the visor.

It will be appreciated that various means can be provided for supporting the visor on a wearer's head. For example, in one embodiment, a head support means comprises a pair of arms 20, 22 that can be joined at overlapping end portions 24, 26. The end portions of the arms may be brought around the back of the wearer's head and fastened to together in such a way as to adjust to the head size of the wearer. In one embodiment, the arms can be shorter so that the ends do not overlap and an elastic band that extends from the end portion of one arm to the end portion of the other arm can be used instead of joining overlapping end portions as described above. In other words, the support means can be a combination of arms and an elastic band, strap or other stretchable mechanism. In other embodiments the length of the arms can vary, and each arm can have a different length in relation to the other arm. For example, the support means can comprising a long arm that extends from one side of the visor, wraps around the wearer's head, and connects to short "stub" arm on the other side of the visor.

In one embodiment, the end portions of the arms are connected together using connection means such as hook and loop fastener strips 28, 30. It will be appreciated, however, that other connection means such as hooks, snaps, buckles, adhesive strips, ties, buttons, tabs and slots, and other connectors known to those skilled in the art can be used. In the preferred embodiment the connection means is reversible so that the end portions of the arms can be joined and separated as desired.

Also, in one embodiment a forehead support member 32 is provided. The forehead support member is preferably made from a rigid or semi-rigid material and has a rearward facing surface 34 to which is attached a cushion 36. The cushion may be made of, for example, a soft rubberized or foam material that will provide a comfortable surface that rests against the user's forehead (not shown). The cushion is preferably attached to using an adhesive material. Alternatively the forehead support member can be made from a soft rubberized material or the like that provides an integral cushion.

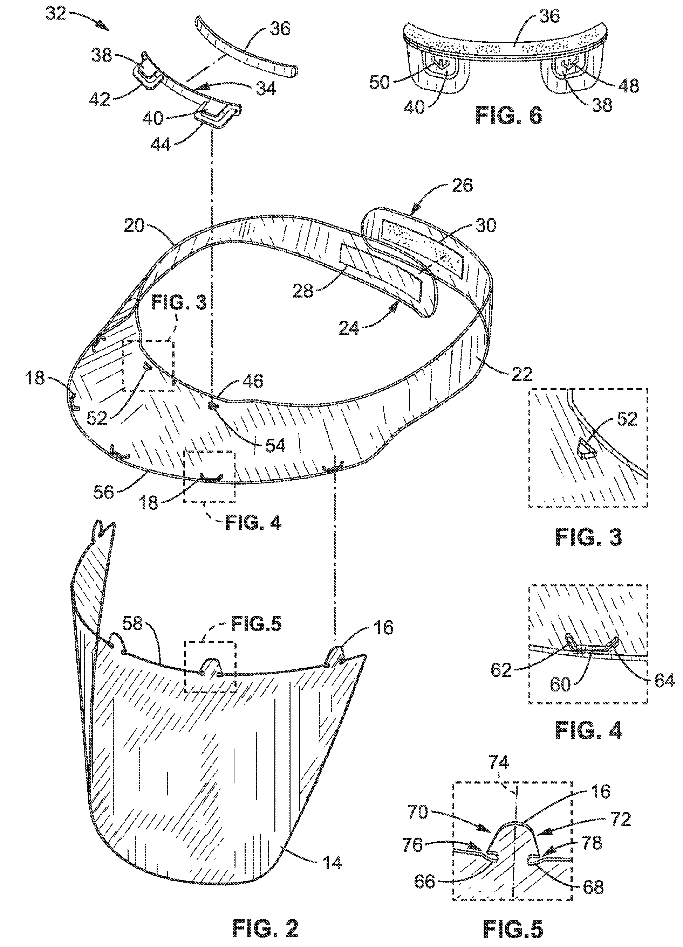

Referring more specifically to FIG. 2, FIG. 3 and FIG. 6 together, in one embodiment the forehead support member includes a pair of forward-facing upper tabs 38, 40 spaced apart from lower frames 42, 44, respectively. The spaces between the upper tabs and lower frames are configured to receive portions of the rear edge 46 of the visor such that the edge of the visor slides into the spaces and the tabs and frames slide over the edge of the visor. To prevent movement and secure the forehead support member in place in relation to the visor, the undersides of the tabs include protrusions 48, 50 (here shown as arrow-shaped) that fit into and mate with corresponding cutouts 52, 54 (here shown as triangular-shaped). It will be appreciated that the protrusions and cutouts could have other shapes as well and perform the same or substantially similar function.

For purposes of this description, it will be appreciated that terms such as "upper", "lower", "forward", "rearward", "outward", "inward", "front", "back", "top", "bottom", and "side" may be used to denote relative orientations, such as in the context of an assembled apparatus or when the apparatus is positioned on the wearer's head, and are not positional requirements per se.

Referring more particularly to FIG. 2, FIG. 4 and FIG. 5, in one embodiment the face shield is secured to the visor using an innovative tab and slot mechanism. In one embodiment, the visor has a forward edge 56 and a plurality of slots 16 positioned along the edge and inward of the edge. In the embodiment shown, five slots are provided but the number of slots can vary. In one embodiment, the face shield has an upper edge 58 and a plurality of tabs 16 that extend from the upper edge of the face shield. In the embodiment shown, five tabs are provided to correspond to the number of slots but the number of tabs can vary as the number of corresponding tabs vary.

The tabs and slots are configured such that a tab can easily be bent and inserted into a corresponding slot and, after, the tab is inserted, the tab automatically springs back to its original flat position to secure the face shield to the visor. It will be appreciated that a tab can be "bent" by any form of deformation that changes its shape from flat to arcuate, such as, for example, by the user bending the tab with his or her fingers or sliding the flat tab into the slot wherein the shape of the slot bends the tab. As illustrated in FIG. 4 this is facilitated by, in one embodiment, configuring the slots such that they have slots have a straight central portion 60 and angled portions 62, 64 positioned at each end of the straight central portion.

As illustrated in FIG. 5, in one embodiment, the upper edge of the face shield is partially "undercut" beneath each tab. Undercuts 66, 68 span from the sides 70, 72 of a tab toward the central axis 74 through the tab such that the tab appears "tree-shaped" with the undercuts forming a pair of "notches" 76, 78 in the face shield positioned beneath each tab. In one embodiment (not shown) the notch is beneath a tab on only side of the tab. In another embodiment, some tabs can have both notches and some tabs can have only one notch.

In one embodiment (not shown) there is a slit or a notch in the tab that extends along the central axis from the tip of the tab toward the upper edge of the face shield, whereby "ears" are formed that assist with tab insertion. The slit or notch need not extend the entire distance from the tip of the tab to the edge of the visor.

It will be appreciated that the above-described configuration of slots and tabs facilitates greater ease of tab insertion and assembly of the apparatus and, further, provides for a more secure connection between the visor and the face shield.

In one embodiment the arms may include an arcuate recess 80 in the area that will rest above the wearer's ears. The arms may be formed in various widths, lengths, and other dimensions. The visor and face shield may be formed in various dimensions as well.

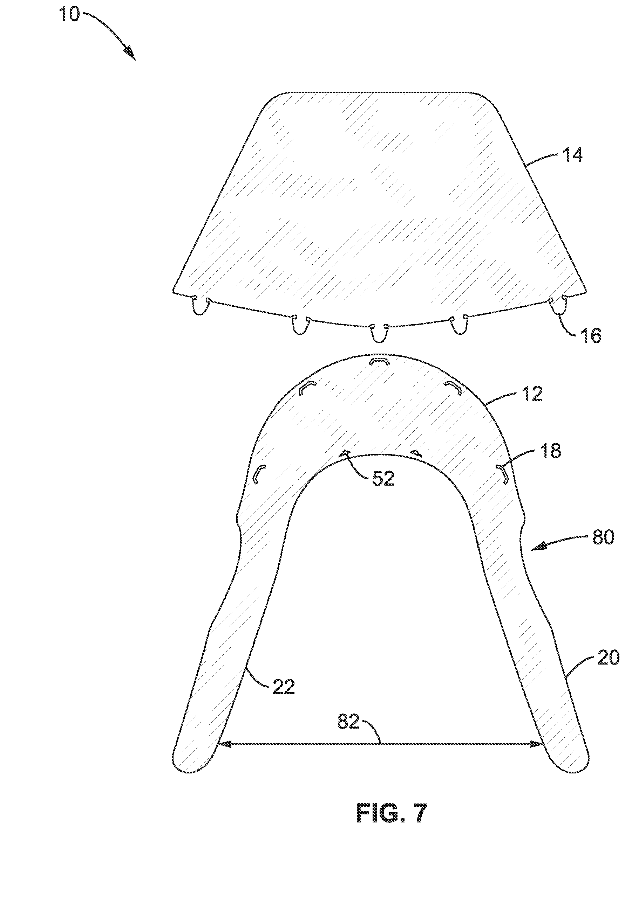

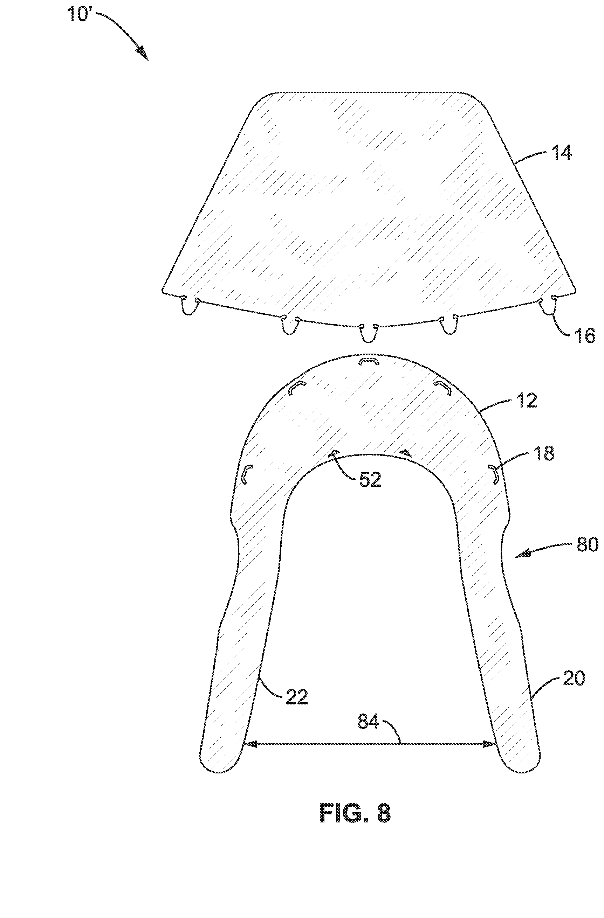

In one embodiment the face shield and visor are formed as components that are initially flat as illustrated in FIG. 7 and FIG. 8. The visor and face shield can be, for example, fabricated by stamping from flat sheets of material, thus avoiding more expensive techniques such as injection molding and various fabricating techniques. The flat components are then assembled by folding, twisting and/or bending as required.

In various embodiments, the spread of the arms can vary. FIG. 7 illustrates an example of a wider spread 82 in relation to a narrower spread 84 illustrated in FIG. 8. An advantage of a wider spread is that the forehead support member is more elevated and the slope of the visor (forehead support member to the front edge of visor) has a greater angle than with a narrow spread such the visor provides more shade over the wearer's eyes. The wider the spread, the more pronounced the downward slope.

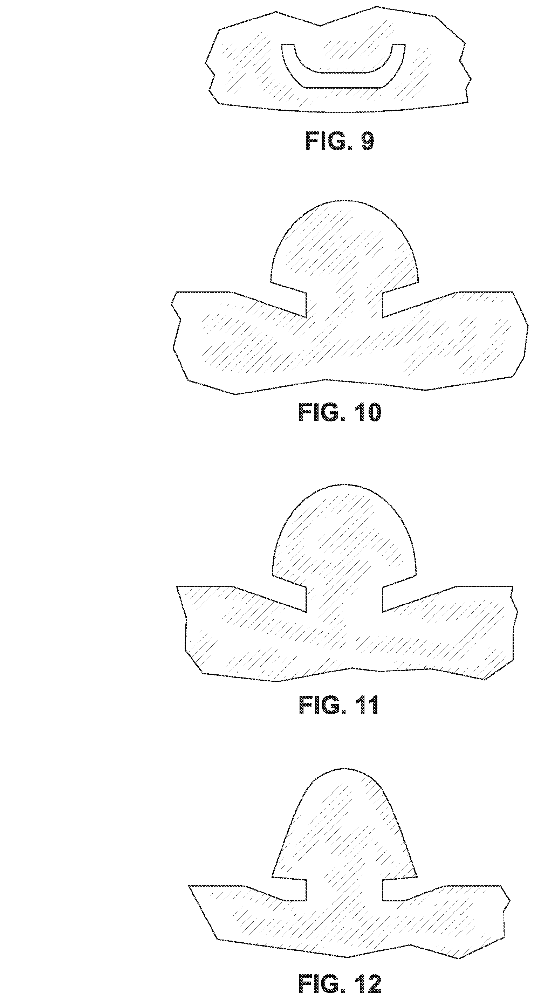

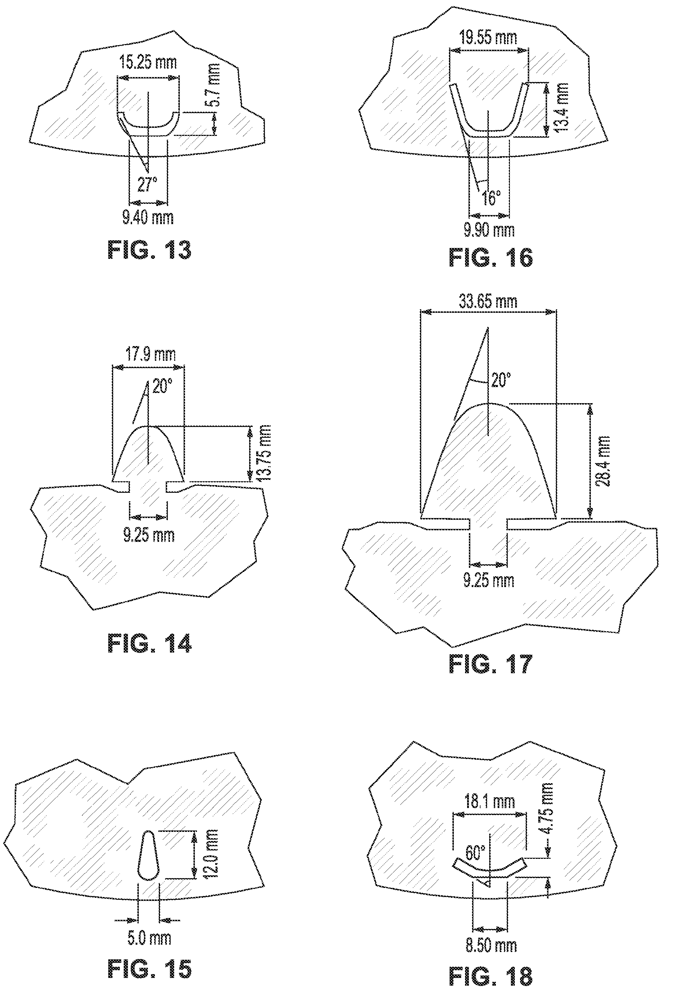

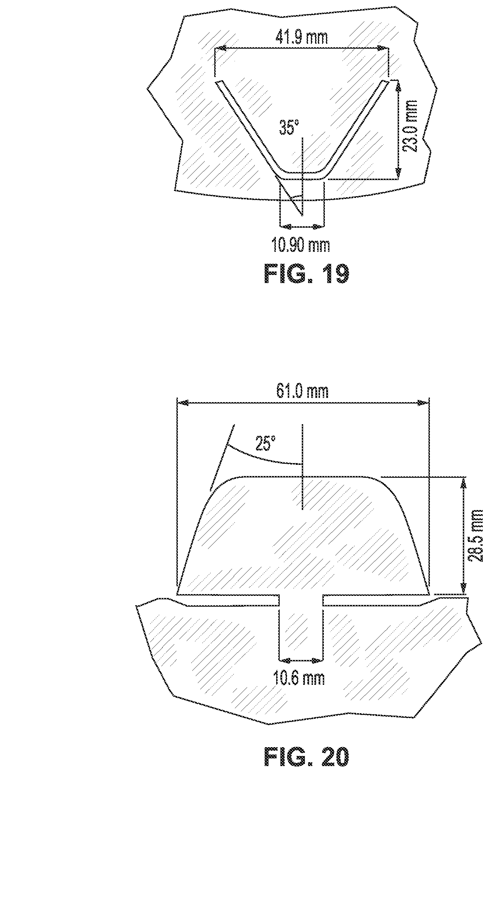

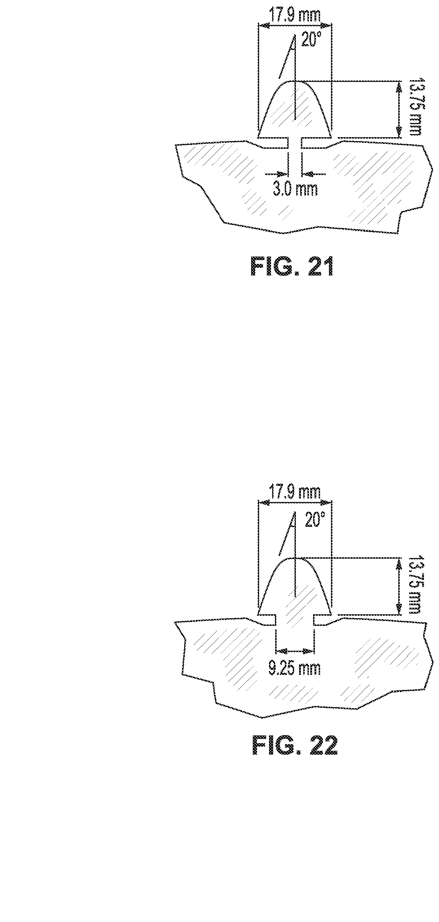

It will be appreciated that the shapes of the tabs and slots may vary. FIG. 9 illustrates a slot with ends that are slightly more rounded than shown in FIG. 4. The important feature of the slots are that they are not merely straight from end to end but have angled portions at each of a central straight portion as previously described. FIG. 10 through FIG. 12 illustrate that the shapes of the tabs may vary as well, with "mushroom-shaped" tabs of different sizes illustrated in FIG. 10 and FIG. 11 and a "tree-shaped" tab illustrated in FIG. 12. FIG. 13 through FIG. 22 illustrate various shapes and provide sample dimensions of slots and tabs.

In all embodiments, when the apparatus is assembled and placed on the wearer's head, the visor preferably supports the face shield and the face shield extends downward from the visor. The face shield also preferably extends down to below the level of the wearer's mouth and around the sides of the wearer's face, thus providing superior, frontal and lateral protection from contaminants. It will be appreciated that a face shield that is supported by a visor and which extends downward therefrom when assembled or worn can also be said to "depend" from the visor.

The visor and face shield can be formed from various synthetic materials such as plastic, cross-linked polycarbonate, mylar, acetate or other material that is flexible. The thickness of the material can also vary to control flexibility, resilience, and strength. The visor may be made transparent or in various colors or tints. The visor may also carry ornamental graphics or text which are applied to the visor like decals, or stamped, or printed, or silkscreened, or applied onto the visor in conventional ways. The face shield is preferably transparent but can be tinted or colored, and may also carry ornamental graphics or text, provided that the wearer's vision is not obscured to an undesirable degree.

### B. Univisor

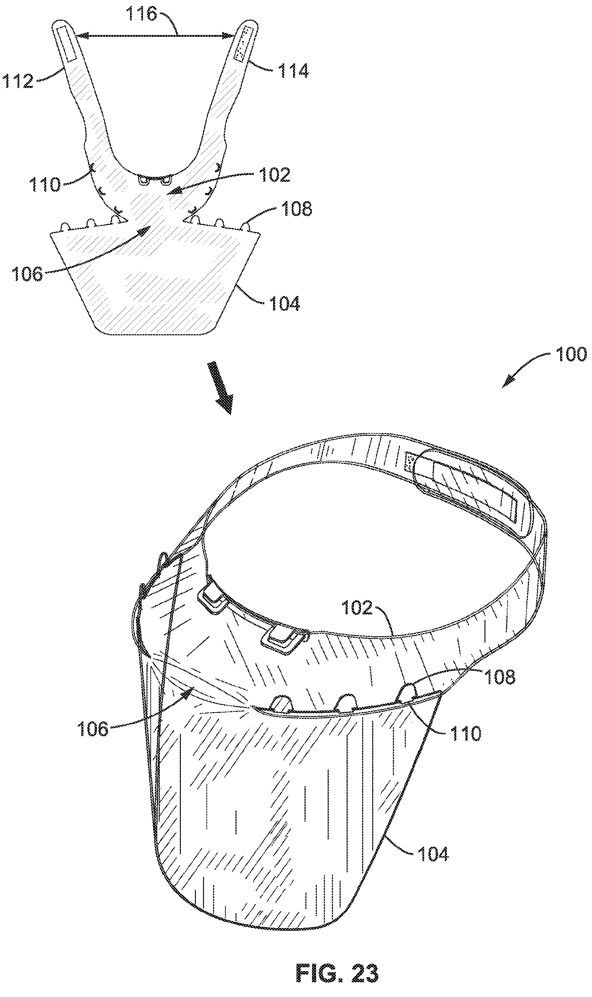

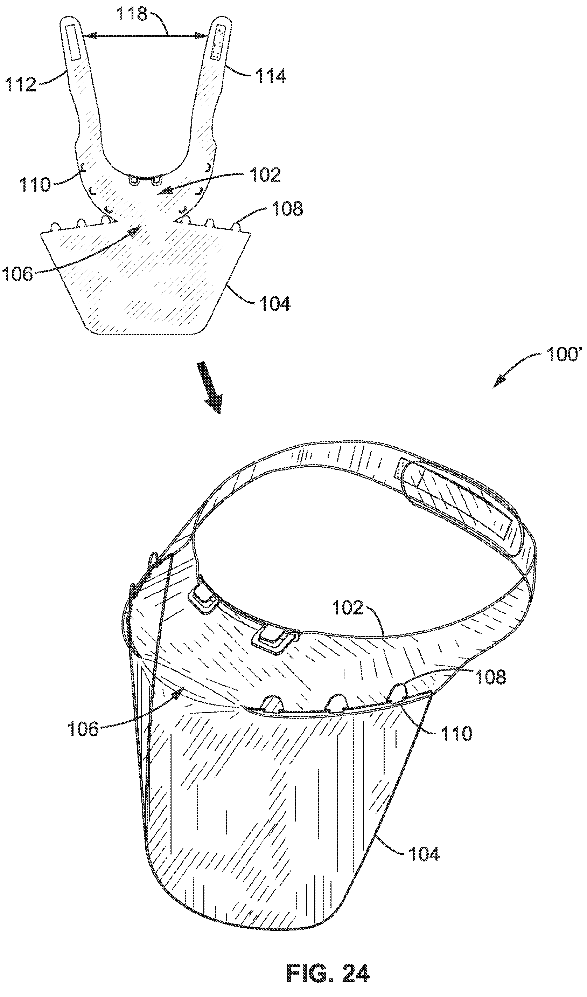

FIG. 23 and FIG. 24 illustrate an embodiment of a visor and protective face shield apparatus 100 that is formed from a unitary flat blank instead of using separate components as described above. To assemble the apparatus, the face shield is bent with respect to the visor and secured to the visor using the previously-described improved tabs and slots such that the visor supports the face shield.

More particularly, in one embodiment, assembly involves bending the visor portion 102 at an approximate right angle in relation to the face shield portion 104 along a "hinge" area 106. Thereafter, tabs 108 are inserted through corresponding slots 110. In the embodiment shown, six tabs and slots are used but the number can vary.

In various embodiments, the spread of the arms 112, 114 can vary. FIG. 23 illustrates an example of a wider spread 116 in relation to a narrower spread 118 illustrated in FIG. 24. In other respects, the apparatus 100 is the same as apparatus 10 described above.

### C. Visor and Face Shield Improvements

It will be appreciated from the foregoing that the slots and tabs described herein can be used with any visor and protective face shield apparatus the face shield needs to be secured to the visor. Accordingly, in one embodiment, an improvement to a visor and face shield apparatus comprises a plurality of slots spaced apart along an edge of a visor and positioned inward in relation to the edge of the visor; and a plurality of tabs spaced apart along an edge of a face shield and extending outward from the edge of the face shield, said plurality of tabs corresponding to said plurality of slots;wherein each said slot has a substantially straight central portion and angled end portions extending from the central portion; wherein the edge of the face shield is undercut in areas beneath each side of a tab to form a notch beneath each side of the tab; wherein each tab is insertable into a corresponding slot by bending the tab from a flat position to an arcuate position; and wherein each tab automatically springs back to the flat position after insertion, whereby the visor and face shield are secured together.

### D. Slot and Tab Joinery Method

It will further be appreciated that an improved joinery method has been developed. In one embodiment the method comprises providing a plurality of slots spaced apart along an edge of a first component and inward in relation to the edge of the first component; providing a plurality of tabs spaced apart along an edge of a second component and extending outward from the edge of the second component, said plurality of tabs corresponding to said plurality of slots; wherein each said slot has a substantially straight central portion and angled end portions extending from the central portion; wherein the edge of the second component is undercut in areas beneath each side of a tab to form a notch beneath each side of the tab; and bending each tab from a flat position to an arcuate position and inserting the tab into a corresponding slot; wherein each tab automatically springs back to the flat position after insertion, whereby the first and second components are secured together.

Therefore, from the description herein it will be appreciated that the present disclosure encompasses multiple implementations which include, but are not limited to, the following:

A visor and protective face shield apparatus according to the present invention comprises:
(a) a visor having a plurality of slots spaced apart along an edge of the visor and positioned inward in relation to the edge of the visor, each said slot having a substantially straight central portion and angled end portions extending from the central portion; and (b) a face shield having a plurality of tabs spaced apart along an edge of the face shield and extending from the edge of the face shield, said plurality of tabs corresponding to said plurality of slots; (c) wherein the edge of the face shield is undercut in an area beneath each tab to form a notch beneath a side of the tab; (d) wherein each tab is insertable into a corresponding slot by the tab bending from a flat position to an arcuate position; and (e) wherein each tab automatically springs back to the flat position after insertion.

As used herein, the term "set" refers to a collection of one or more objects. Thus, for example, a set of objects can include a single object or multiple objects.

As used herein, the terms "approximately", "approximate", "substantially" and "about" are used to describe and account for small variations. When used in conjunction with an event or circumstance, the terms can refer to instances in which the event or circumstance occurs precisely as well as instances in which the event or circumstance occurs to a close approximation. When used in conjunction with a numerical value, the terms can refer to a range of variation of less than or equal to ± 10% of that numerical value, such as less than or equal to ±5%, less than or equal to ±4%, less than or equal to ±3%, less than or equal to ±2%, less than or equal to ±1 %, less than or equal to ±0.5%, less than or equal to ±0.1 %, or less than or equal to ±0.05%. For example, "substantially" aligned can refer to a range of angular variation of less than or equal to ±10°, such as less than or equal to ±5°, less than or equal to ±4°, less than or equal to ±3°, less than or equal to ±2°, less than or equal to ±1°, less than or equal to ±0.5°, less than or equal to ±0.1°, or less than or equal to ±0.05°.

Additionally, amounts, ratios, and other numerical values may sometimes be presented herein in a range format. It is to be understood that such range format is used for convenience and brevity and should be understood flexibly to include numerical values explicitly specified as limits of a range, but also to include all individual numerical values or sub-ranges encompassed within that range as if each numerical value and sub-range is explicitly specified. For example, a ratio in the range of about 1 to about 200 should be understood to include the explicitly recited limits of about 1 and about 200, but also to include individual ratios such as about 2, about 3, and about 4, and sub-ranges such as about 10 to about 50, about 20 to about 100, and so forth.

Although the description herein contains many details, these should not be construed as limiting the scope of the disclosure but as merely providing illustrations of some of the presently preferred embodiments.

## Claims

1. A visor and protective face shield apparatus (10), the apparatus comprising:
(a) a visor (12) having a plurality of slots (18) spaced apart along an edge of the visor and positioned inward in relation to the edge of the visor, each said slot having a substantially straight central portion and angled end portions extending from the central portion; and
(b) a face shield (14) having a plurality of tabs (16) spaced apart along an edge of the face shield and extending from the edge of the face shield, said plurality of tabs corresponding to said plurality of slots, **characterized in that**,
(c) the edge (58) of the face shield is undercut (66, 68) in an area beneath each tab (16) to form a notch (76, 78) beneath a side of the tab;
(d) wherein each tab (16) is insertable into a corresponding slot (18) by the tab bending from a flat position to an arcuate position; and
(e) wherein each tab automatically springs back to the flat position after insertion.

2. The apparatus of claim 1, wherein the edge (58) of the face shield (14) is undercut in areas beneath each side of a tab (16) to form a notch (76, 78) beneath each side of the tab.

3. The apparatus of claim 1, further comprising means (20, 22) for supporting the visor on a wearer's head.

4. The apparatus of claim 1, further comprising a forehead support member (32) that is attachable to the visor (12).

5. The apparatus of claim 1, wherein the tabs (16) and slots (18) secure the face shield (14) together such that the face shield depends from the visor when worn.

6. The apparatus of claim 1, wherein the visor (12) and face shield (14) are separate components.

7. The apparatus of claim 1, wherein the visor (12) and face shield (14) are a unitary component.

8. The apparatus of claim 1, wherein the visor (12) includes an ornamental graphic image.

9. The apparatus of claim 1, wherein the face shield and visor are secured together by the tabs and slots and wherein the face shield (14) depends from the visor (12) when worn.

10. A method of joining a visor with a protective face shield, the method comprising:
providing a plurality of slots (18) spaced apart along an edge (56) of a first component and inward in relation to the edge of the first component;
providing a plurality of tabs (16) spaced apart along an edge (58) of a second component and extending outward from the edge of the second component, said plurality of tabs corresponding to said plurality of slots;
wherein each said slot has a substantially straight central portion and angled end portions extending from the central portion;
wherein the edge of the second component is undercut (66, 68) in an area beneath each tab to form a notch beneath a side of the tab;
bending each tab (16) from a flat position to an arcuate position and inserting the tab into a corresponding slot (18);
wherein each tab automatically springs back to the flat position after insertion, whereby the first and second components are secured together.

11. The method of claim 10, wherein the edge of the second component is undercut (66, 68) in areas beneath each side of a tab (16) to form a notch (76, 78) beneath each side of the tab.

## Patentansprüche

1. Visier und Gesichtsschutzvorrichtung (10), wobei die Vorrichtung folgendes umfasst:
(a) ein Visier (12) mit einer Mehrzahl von Schlitzen (18), die entlang einer Kante des Visiers mit Zwischenabständen angeordnet und im Verhältnis zu der Kante des Visiers einwärts positioniert sind, wobei jeder Schlitz ein im Wesentlichen gerades zentrales Teilstück aufweist sowie angewinkelte Endstücke, die sich von dem zentralen Teilstück erstrecken; und
(b) einen Gesichtsschutz (14) mit einer Mehrzahl von Streifen (16), die entlang einer Kante des Gesichtsschutz mit Zwischenabständen angeordnet sind und sich von der Kante des Gesichtsschutzes erstrecken, wobei die Mehrzahl von Streifen der Mehrzahl von Schlitzen entspricht,
**dadurch gekennzeichnet, dass**
(c) die Kante (58) des Gesichtsschutzes in einem Bereich unterhalb jedes Streifens (16) unterschnitten ist, so dass unter einer Seite des Streifens eine Kerbe (76, 78) gebildet wird;
(d) wobei jeder Streifen (16) in einen entsprechenden Schlitz (18) eingeführt werden kann, indem der Streifen aus einer flachen Stellung an eine gebogene Stellung gebogen wird; und
(e) wobei jeder Streifen nach dem Einführen automatisch an die flache Stellung zurückspringt.

2. Vorrichtung nach Anspruch 1, wobei die Kante (58) des Gesichtsschutzes (14) in Bereichen unterhalb jeder Seite eines Streifens (16) unterschnitten ist, so dass eine Kerbe (76, 78) unterhalb jeder Seite des Streifens gebildet wird.

3. Vorrichtung nach Anspruch 1, wobei diese ferner Mittel (20, 22) zum Stützen des Visiers an dem Kopf eines Trägers umfasst.

4. Vorrichtung nach Anspruch 1, wobei diese ein Stirnstützelement (32) umfasst, das an dem Visier (12) angebracht werden kann.

5. Vorrichtung nach Anspruch 1, wobei die Streifen (16) und Schlitze (18) den Gesichtsschutz (14) zusammenhalten, so dass der Gesichtsschutz beim Tragen an dem Visier gehalten wird.

6. Vorrichtung nach Anspruch 1, wobei es sich bei dem Visier (12) und dem Gesichtsschutz (14) um getrennte Komponenten handelt.

7. Vorrichtung nach Anspruch 1, wobei das Visier (12) und der Gesichtsschutz (14) eine unitäre Komponente sind.

8. Vorrichtung nach Anspruch 1, wobei das Visier (12) ein verzierendes grafisches Bild aufweist.

9. Vorrichtung nach Anspruch 1, wobei der Gesichtsschutz und das Visier durch die Streifen und Schlitze zusammengehalten werden, und wobei der Gesichtsschutz (14) beim Tragen an dem Visier (12) gehalten wird.

10. Verfahren zur Verbindung eines Visiers mit einem Gesichtsschutz, wobei das Verfahren folgendes umfasst:
Bereitstellen einer Mehrzahl von Schlitzen (18), die entlang einer ersten Komponente mit Zwischenabständen angeordnet und im Verhältnis zu der Kante der ersten Komponente einwärts positioniert sind,
Bereitstellen einer Mehrzahl von Streifen (16), die entlang einer Kante einer zweiten Komponente mit Zwischenabständen angeordnet sind und sich von der Kante der zweiten Komponente erstrecken, wobei die Mehrzahl von Streifen der Mehrzahl von Schlitzen entspricht,
wobei jeder Schlitz ein im Wesentlichen gerades zentrales Teilstück aufweist sowie angewinkelte Endstücke, die sich von dem zentralen Teilstück erstrecken;
wobei die Kante der zweiten Komponente in einem Bereich unterhalb jedes Streifens unterschnitten (66, 68) wird, so dass unter einer Seite des Streifens eine Kerbe gebildet wird;
wobei jeder Streifen (16) aus einer flachen Stellung an eine gebogene Stellung gebogen wird, und wobei der Streifen in einen entsprechenden Schlitz (18) eingeführt wird;
wobei jeder Streifen nach dem Einführen automatisch an die flache Stellung zurückspringt, wodurch die erste und die zweite Komponente aneinander gesichert werden.

11. Verfahren nach Anspruch 10, wobei die Kante der zweiten Komponente in Bereichen unterhalb jeder Seite eines Streifens (16) unterschnitten (66, 68) wird, so dass eine Kerbe (76, 78) unterhalb jeder Seite des Streifens gebildet wird.

## Revendications

1. Appareil formant visière et écran facial de protection (10), l'appareil comprenant :
(a) une visière (12) comportant plusieurs fentes (18) espacées le long d'un bord de la visière et positionnées vers l'intérieur par rapport au bord de la visière, chacune desdites fentes ayant une partie centrale sensiblement droite et des parties d'extrémité angulaires s'étendant à partir de la partie centrale ; et
(b) un écran facial (14) comportant une pluralité de languettes (16) espacées le long d'un bord de l'écran facial et s'étendant à partir du bord de l'écran facial, ladite pluralité de languettes correspondant à ladite pluralité de fentes,
**caractérisé en ce que**,
(c) le bord (58) de l'écran facial est évidé (66, 68) dans une zone sous chaque languette (16) pour former une encoche (76, 78) sous un côté de la languette ;
(d) chaque languette (16) pouvant être insérée dans une fente (18) correspondante par la flexion de la languette d'une position plate à une position arquée ; et
(e) chaque languette revenant automatiquement en position plate après l'insertion.

2. Appareil selon la revendication 1, le bord (58) de l'écran facial (14) étant évidé dans des zones sous chaque côté d'une languette (16) pour former une encoche (76, 78) sous chaque côté de la languette.

3. Appareil selon la revendication 1, comprenant en outre un moyen (20, 22) pour soutenir la visière sur la tête d'un porteur.

4. Appareil selon la revendication 1, comprenant en outre un élément de support de front (32) qui peut être fixé à la visière (12).

5. Appareil selon la revendication 1, les languettes (16) et les fentes (18) fixant l'écran facial (14) ensemble de sorte que l'écran facial soit suspendu à la visière lorsqu'il est porté.

6. Appareil selon la revendication 1, la visière (12) et l'écran facial (14) étant des composants séparés.

7. Appareil selon la revendication 1, la visière (12) et l'écran facial (14) étant un composant unitaire.

8. Appareil selon la revendication 1, la visière (12) comprenant une image graphique ornementale.

9. Appareil selon la revendication 1, l'écran facial et la visière étant fixés ensemble par les languettes et les fentes et l'écran facial (14) étant suspendu à la visière (12) lorsqu'il est porté.

10. Procédé d'assemblage d'une visière à un écran facial de protection, le procédé comprenant les étapes consistant à :
fournir une pluralité de fentes (18) espacées le long d'un bord (56) d'un premier composant et vers l'intérieur par rapport au bord du premier composant ;
fournir une pluralité de languettes (16) espacées le long d'un bord (58) d'un second composant et s'étendant vers l'extérieur du bord du second composant, ladite pluralité de languettes correspondant à ladite pluralité de fentes ;
chacune desdites fentes ayant une partie centrale sensiblement droite et des parties d'extrémité angulaires s'étendant à partir de la partie centrale ;
le bord du second composant étant évidé (66, 68) dans une zone sous chaque languette pour former une encoche sous un côté de la languette ;
plier chaque languette (16) d'une position plate à une position arquée et insérer la languette dans une fente (18) correspondante ;
chaque languette revenant automatiquement en position plate après insertion, moyennant quoi les premier et second composants sont fixés ensemble.

11. Procédé selon la revendication 10, le bord du second composant étant évidé (66, 68) dans des zones sous chaque côté d'une languette (16) pour former une encoche (76, 78) sous chaque côté de la languette.
